Europäisches Patentamt

European Patent Office

Office européen des brevets

(11) Veröffentlichungsnummer: **0 268 575**
**A2**

## (12) EUROPÄISCHE PATENTANMELDUNG

(21) Anmeldenummer: 87890253.5

(22) Anmeldetag: 11.11.87

(51) Int. Cl.⁴: **C 12 N 9/96**
**A 23 K 1/16, A 23 K 1/00**

(30) Priorität: 17.11.86 AT 3054/86

(43) Veröffentlichungstag der Anmeldung:
25.05.88 Patentblatt 88/21

(84) Benannte Vertragsstaaten: **ES FR**

(71) Anmelder: **Agrocon Agrar-Consulting Gesellschaft m.b.H.**
**Hafferlstrasse 7/7**
**A-4020 Linz (AT)**

(72) Erfinder: **Lenauer, Peter**
**Ertlerstrasse 10**
**A-3352 St. Peter/Au (AT)**

(74) Vertreter: **Pawloy, Heinrich, Dr. et al**
**Riemergasse 14**
**A-1010 Wien (AT)**

(54) **Mittel zur Aktivierung fermentativer Prozesse sowie Verfahren zum Beschleunigen des fermentativen Abbaues von Naturstoffen.**

(57) Mittel, die Fermente, insbesondere Glykosidase, Cellulase, Proteinase, Lipase oder Amylase, und Saponin, wie ein Triterpen-Saponin, insbesondere Quillaja-Saponin, enthalten, aktivieren fermentative Prozesse und beschleunigen den fermentativen Abbau von Naturstoffen, insbesondere ligninhaltigen Polysacchariden.

EP 0 268 575 A2

# 0 268 575

**Beschreibung**

### Mittel zur Aktivierung fermentativer Prozesse sowie Verfahren zum Beschleunigen des fermentativen Abbaues von Naturstoffen

Fermentative Prozesse werden durch Enzyme gesteuert, die ihrerseits durch biologische Vorgänge hergestellt werden. So werden beispielsweise Glykosidasen, durch welche die Glykosidbindungen in Oligo- bzw. Polysacchariden aufgespaltet werden, sowohl im Verdauungstrakt von tierischen Körpern als auch durch Mikroorganismen produziert.

Es ist bereits bekannt, auf biologischem Wege, nämlich durch Einsatz von Mikroorganismen, gewonnene Glykosidase, insbesondere Cellulase, in sich schwer verdaulichen Futtermitteln zuzusetzen, um die Verwertbarkeit dieser Futtermittel durch das betreffende Tier zu verbessern. Weiterhin ist es bereits bekannt, hochzellulosehaltiges Futtersubstrat, beispielsweise Stroh, Ölpreßkuchen aus Sonnenblumenkernen oder dgl. vor der Verfütterung mit Cellulase zu behandeln, um dadurch einerseits die im Tiermagen schwer oder überhaupt nicht verdaulichen Celluloseanteile aufzuschließen und andererseits durch diesen Aufschluß auch Nährsubstanzen, wie beispielsweise verschiedene Proteine, freizusetzen, die sonst durch chemischen oder mechanischen Einbau in die Cellulose durch den tierischen Verdauungsvorgang nicht verwertet werden konnten.

Ziel der vorliegenden Erfindung ist es nun, nicht nur derartige an sich bekannte Verfahrensweisen zu beschleunigen bzw. in der Wirksamkeit zu verbessern, sondern darüberhinaus außerdem ein Mittel zu schaffen, welches ganz allgemein fermentative Prozesse aktiviert und somit nicht nur zum Aufschluß von Cellulose für die Herstellung von Futtermitteln, sondern darüberhinaus zur Lösung verschiedener anderer Probleme eingesetzt werden kann.

Hochsaponinhaltige Naturstoffe werden bereits seit geraumer Zeit als Futtermittel verwendet; diesbezüglich sei insbeson dere an die Verfütterung von Roßkastanien bzw. Luzerne verwiesen, welches beides Pflanzen bzw. Pflanzenprodukte sind, die einen hohen Saponingehalt aufweisen. Saponine an sich sind eine Verbindungsgruppe, denen eine Reihe von Eigenschaften gemeinsam ist. Ihrer chemischen Struktur nach sind die Saponine Glykoside, deren Aglyka, die Sapogenine, verschiedene Grundstrukturen aufweisen, auf Grund derer man die Saponine in drei Klassen, die Triterpen-Saponine, die neutralen Steroid-Saponine und die basischen Steroid-Saponine einteilt. Während die letzteren beiden Arten an sich wenig wirtschaftliche Bedeutung besitzen und, wenn überhaupt verwendet, in erster Linie als Rohstoff, zur Herstellung verschiedener anderer Substanzen, beispielsweise Pharmaka, dienen, werden die Triterpen-Saponine technisch auf Grund ihrer Oberflächenaktivität als solche angewendet. Quillaja-Extrakt, ein triterpen-saponinhaltiger Extrakt aus der Quillajarinde, ist von der FDA/USA zur Verwendung in Lebensmitteln und Getränken zugelassen. Er wird im Orient außerdem zur Herstellung von Süßspeisen eingesetzt.

Saponine wurden weiterhin auch schon auf landwirtschaftlichem Gebiet eingesetzt, beispielsweise als Aufbereitungsmittel für Abfallprodukte (EP-A1 0 063 621) sowie als Aufbereitungsmittel für land- und forstwirtschaftliche Böden (EP-A1 0 063 622). In beiden Fällen soll durch den Zusatz bzw. die Aufbringung von saponinhaltigen wässerigen Lösungen das aerobe Bakterienwachstum gegenüber dem anaeroben Bakterienwachstum begünstigt und dadurch der bakterielle Aufschluß der Abfallprodukte einerseits bzw. der Böden andererseits beschleunigt werden. Weiterhin wird angenommen, daß bei dem dort beschriebenen Verfahren auch das Wachstum der anaeroben Bakterien gehemmt wird. In der erstgenannten EP-A1 wird weiterhin erwähnt, daß Saponine auch zur Aufbereitung von nährstoffhaltigen landwirtschaftlichen Nebenprodukten eingesetzt werden können, wobei als derartige Nebenprodukte Stroh, insbesondere Maisstroh, oder Reisschalen oder dgl. erwähnt werden, die unter Zusatz von Saponinen kompostiert werden sollen, durch welche Kompostierung zum Teil wertvolle Futtermittel erhalten werden sollen. Auch in diesem Fall soll die Kompostierung durch Bakterienwachstum erfolgen und es soll eine erhebliche Beschleunigung des Verrottungsprozesses erzielt werden. Für diese spezielle Kompostierung zur Herstellung von Futtermitteln wird ausdrücklich auf die Anwesenheit von Melasse verwiesen, welche einen Nährboden für die aeroben Bakterien bildet und durch ihre Klebrigkeit dafür sorgen soll, daß die Saponine an den Feststoffen haften bleiben und nicht ohne weiteres entfernt werden können.

Obwohl durch dieses dort vorgeschlagene Verfahren zweifellos eine Beschleunigung der natürlichen Verrottung durch Bakterien erfolgen kann, verlaufen bakteriell induzierte Prozesse immer noch relativ langsam, so daß man, wie schon eingangs erwähnt, zur Beschleunigung dieser natürlichen Vorgänge die durch das relativ langsame Wachstum der Bakterien gebildeten wirksamen Fermente zunächst isoliert und diese isolierten Fermente dann erst zum eigentlichen Abbauverfahren, insbesondere bei der Herstellung von Futtermitteln aus landwirtschaftlichen Abfallprodukten, eingesetzt hat. Derartige Verfahren, bei welchen die gewünschten Fermente direkt eingesetzt werden, verlaufen erstens selektiver und zweitens wesentlich schneller, da bei diesen Verfahren der notwendige Fermentanteil a priori direkt eingesetzt werden kann und nicht erst gewartet werden muß, bis durch mehr oder weniger zufälliges Bakterienwachstum (auch wenn dieses irgendwie beschleunigt werden kann) das gewünschte Ferment im notwendigen Anteil gebildet wird.

Ganz allgemein kann somit gesamt werden, daß insbesondere bei der Herstellung von Futtermitteln aus Abfallstoffen aus ökonomischer Sicht ein Verfahren vorzuziehen ist, bei welchem das jeweilige Abfallprodukt, wie beispielsweise Stroh, Sonnenblumenschrot, Kleie oder dgl., für die bessere Verwertung durch den Tiermagen aufgeschlossen werden soll.

Es wurde nun überraschenderweise festgestellt, daß Saponine, insbesondere jedoch Triterpen-Saponine, wobei vor allem Quillaja-Saponin in Frage kommt, die Fähigkeit besitzen, die Abbaugeschwindigkeit hochmolekularer Stoffe durch Fermente beträchtlich zu beschleunigen. So konnte festgestellt werden, daß der Aufschlußgrad von Cellulose, wenn diese in an sich bekannter Weise mit Cellulasefermenten aufgeschlossen wird, beträchtlich verbessert wird, wenn zusätzlich beim Aufschlußverfahren Saponine zugesetzt werden. Analoge Resultate werden auch hinsichtlich der Abbaugeschwindigkeit anderer Fermente, wie Proteasen und Lipasen, erzielt.

Gegenstand der vorliegenden Erfindung daher ein Mittel zur Aktivierung fermentativer Prozesse, welches dadurch gekennzeichnet ist, daß es neben Konservierungs- bzw. Stabilisierungsmitteln sowie gegebenenfalls Verdünnungsmitteln, Farbstoffen, Geruchskorrigentien, Verdickungsmitteln etc. ein oder mehrere Fermente und Saponin enthält. Wie bereits erwähnt, sind für den gewünschten Zweck Saponine allgemein einsetzbar; die besten Resultate werden jedoch erzielt, wenn als Saponin ein Triterpen-Saponin, vorzugsweise Quillaja-Saponin, eingesetzt wird.

Als Fermente für den erfindungsgemäßen Zweck kommen vor allem Glukosidasen in Frage, von welchen als erste Cellulase erwähnt sein soll; der überraschende Effekt der Beschleunigung des Abbauverfahrens tritt jedoch auch ein, wenn andere Glykosidasen, wie beispielsweise Amylase, eingesetzt werden. Ebenso können auch andere Fermente, wie Proteinasen oder Lipasen, eingesetzt werden. Dies ist insbesondere insoweit von Vorteil, als landwirtschaftliche Abfallprodukte meist nicht ausschließlich aus einer bestimmten Substanz, beispielsweise Cellulose, bestehen, sondern in der Grundsubstanz eingebettet auch andere futtermäßig verwertbare Stoffe, wie beispielsweise Protein oder Fette, vorhanden sind. Diese weiteren futtermäßig verwertbaren Stoffe sind zunächst durch die bezüglichen Fermente nicht angreifbar, da sie in der Gerüstsubstanz der Cellulose eingebaut sind; erst nach Abbau der Cellulose durch Cellulase ist es möglich, diese weiteren nährstoffaktiven Substanzen ihrerseits durch Enzymeinwirkung aufzuschließen bzw. abzubauen.

Ein typisches erfindungsgemäßes Mittel enthält beispielsweise pro Liter 2 g Quillaja-Saponin, 500 g Ferment, 5 g Konservierungsmittel und 300 g Haftmittel; als Haftmittel hat sich in diesem Zusammenhang insbesondere Melasse bewährt.

Insbesondere dann, wenn das erfindungsgemäße Mittel längere Zeit gelagert werden soll, ist es zweckmäßig, das Ferment einerseits und die übrigen Bestandteile andererseits getrennt zu lagern, um unerwünschte Reaktionen innerhalb der Mischung, die zur Schwächung der erwünschten Wirkung führen können, zu vermeiden.

Weiterhin bezieht sich die vorliegende Erfindung auf ein Verfahren zum Beschleunigen des fermentativen Abbaues von Naturstoffen, bei welchem den Naturstoffen, insbesondere ligninhaltigen Polysacchariden, Fermente zugesetzt werden, welches dadurch gekennzeichnet ist, daß man außerdem Saponin bzw. Sapogenin oder Saponin bzw. Sapogenin enthaltende Extrakte bzw. Präparate in einem Anteil von 0,2 bis 20 g Saponin bzw. Sapogenin pro Tonne Substrat zusetzt.

Auch bei diesem Verfahren kommen in erster Linie Triterpen-Saponine bzw. insbesondere Quillaja-Saponin, vorzugsweise in Form des Quillajarindenextraktes, in Frage.

Als Fermente können insbesondere auch hier, wie bereits erwähnt, Cellulase, Amylase, Protease und Lipase allein oder in Gemisch miteinander verwendet werden.

Die Obergrenze des Fermentzusatzes wird in erster Linie durch ökonomische Überlegungen bestimmt, da die Fermente ja ihren Preis haben und ab einem bestimmten Fermentanteil die Wirkung nicht mehr wesentlich beschleunigt werden kann.

Das erfindungsgemäße Verfahren kann praktisch bei jeder Temperatur durchgeführt werden, bei welcher fermentative Prozesse an sich ablaufen; es ist jedoch klar, daß auch hinsichtlich der Wahl der Temperatur ein Optimum vorhanden ist, da auch fermentative Prozesse bei Temperaturerhöhung bis zu einer bestimmten Temperatur rascher ablaufen.

Es wird somit zweckmäßig sein, wenn bei verschiedenen Substraten die optimalen Bedingungen hinsichtlich Saponinanteil, Fermentanteil, Verfahrenstemperatur zunächst durch kurze Vorversuche festgelegt werden, bevor der Hauptanteil des Substrates dem erfindungsgemäßen Verfahren unterworfen wird.

Erfindungsgemäß ist es erstmals möglich, die Abfallprodukte ohne aufwendiges Verfahren in hochwertige Futterprodukte umzuwandeln. Da bei dem erfindungsgemäßen Verfahren ja keine Bakterien mehr gezüchtet werden müssen, genügt es, die Mischung aus Fermenten und Saponin dem zu verfütternden Substrat einige Zeit bis unmittelbar vor der Verfütterung zuzusetzen. Während in ersterem Fall je nach dem Zeitraum, der zwischen Zusatz und Verfütterung verstreicht, das Substrat teilweise oder ganz aufgeschlossen wird, wirkt im zweiten Fall, nämlich bei Zusatz des Saponin-Fermentgemisches unmittelbar vor der Verfütterung, das Gemisch als Verdauungshilfe, wobei der Aufschluß des Substrates im Verdauungstrakt des Tieres erfolgt bzw. verbessert wird. Es ist ersichtlich, daß insbesondere durch letztere Möglichkeit gewaltige Einsparungen sowohl an Arbeitszeit als auch an Siloraum erzielt werden können, da bisher - insbesondere beim Einsatz von Bakterienkulturen zum Aufschluß der Abfallprodukte - diese Produkte lange Zeit in speziell dafür vorgesehenen Behältern gelagert werden mußten, bis die zugesetzten Bakterien zunächst genügend Fermente gebildet und da durch den Abbau bzw. die Kompostierung des Abfallproduktes bewirkt haben.

Das erfindungsgemäße Mittel bzw. das erfindungsgemäße Verfahren können jedoch auch für andere Zwecke als zum Aufschließen von Naturstoffen für Futtermittelzwecke eingesetzt werden. Ganz allgemein kann gesagt werden, daß das erfindungsgemäße Mittel bzw. Verfahren immer dann zur Anwendung gelangen kann, wenn aus irgendeinem Grund hochkondensierte Naturstoffe in kleinere chemische Einheiten zerlegt werden sollen. So hat es sich beispielsweise als vorteilhaft herausgestellt, Samen bzw. Saatgut vor dem Aussäen mit dem erfindungsgemäßen Mittel zu behandeln; dadurch werden die hochmolekularen Samenhüllen zumindest teilweise abgebaut und die Keimfähigkeit des Samens wird wesentlich beschleunigt.

Das folgende Beispiel soll den Gegenstand der vorliegenden Erfindung näher erläutern, ohne daß diese darauf beschränkt sein soll.

**Beispiel:** Behandlung von Sommenblumenextraktionsschrot mit einem erfindungsgemäßen, Cellulase enthaltenden Mittel

Es wurden Laborversuche durchgeführt, wobei Sonnenblumenextraktionsschrot mit dem erfindungsgemäßen Mittel und zur Kontrolle nur mit Cellulase oder nur mit Saponin behandelt wurde.

Von allen Mustern wurden wässerige Lösungen hergestellt und die Mehrbildung von Glukose aus der Lignincellulose gemessen. Die einzelnen Muster wurden erstmals 48 h nach der Behandlung des Schrots und in der Folge nach 1 Monat und nach insgesamt 2 Monaten nach der Behandlung untersucht. Nach diesem Zeitraum wurde der behandelte Schrot einzelnen Futtersorten (herkömmliches Standardfutter) zugesetzt und die so behandelten Fut termittel zum Füttern von Kleinferkeln, Läufern (Ferkel mit einem Gewicht zwischen 30 und 60 kg) und Mastschweinen verwendet.

Durch diesen Fütterungsversuch wurden die laboranalytischen Ergebnisse auf die Praxis bezogen; dabei konnten einerseits die Zugabemengen zum Futter erhöht werden (denn derzeit werden auf Grund der schlechten Verdaulichkeit und Mastleistungen nur im Mastschweinefutter maximal 3 % unbehandelter Extraktionsschrot beigemengt); andererseits wurde eine gesteigerte Massezunahme trotz wesentlich höherem prozentuellen Anteil an behandeltem Schrot erreicht.

```
                                                    Glukose Gew.%


    1) Kontrollmuster: unbehandelter Schrot

       nach 48 h                                        0,028 %

       veränderte sich auch nicht während

       der folgenden 2 Monate

    2) nur Cellulase-Enzym 300 I.E./mg Rohfaser

       nach 48 h                                        0,075 %

       nach 1 Monat                                     0,078 %

       nach 2 Monaten                                   0,194 %

    3) 2 %ige Quillaja-Saponinlösung allein

       500 ml/Tonne nach 48 h                           0,100 %

       veränderte sich auch nicht mehr während

       der folgenden 2 Monate

    4) Quillaja-Saponinlösung + Cellulase in

       Dosierung wie oben nach 48 h                     0,081 %

                           nach 1 Monat                 0,196 %

                           nach 2 Monaten               0,608 %


    Fütterungsversuch:

    1) Kleinstferkel

       a) Kontrollfutter ohne Sonnenblumenschrot
```

b) Versuchsfutter mit gemäß Punkt 4) oben behandeltem Schrot

| | a) | b) |
|---|---|---|
| tägliche Gewichtszunahme in g | a) 390 g | b) 410 g |
| Futterverbrauch kg/kg Gewichtszunahme | a) 3,54 kg | b) 3,51 kg |

2) Läufer-Ferkelmast
   a) Kontrollfutter mit 3 Gew.% unbehandeltem Schrot
   b) Versuchsfutter mit 10 Gew.% gemäß Punkt 4) oben behandeltem Schrot

| | a) | b) |
|---|---|---|
| tägliche Gewichtszunahme in g | a) 696 g | b) 804 g |
| Futterverbrauch kg/kg produziertem Fleischgewicht | a) 3,91 kg | b) 2,54 kg |

3) Mastschweine-Endmast
   a) Kontrollfutter mit 3 Gew.% unbehandeltem Schrot
   b) Versuchsfutter mit 12 Gew.% gemäß Punkt 4) oben behandeltem Schrot

| | a) | b) |
|---|---|---|
| tägliche Gewichtszunahme in g | a) 593 g | b) 638 g |
| Futterverbrauch kg/kg produziertem Fleischgewicht | a) 4,82 kg | b) 4,37 kg |

Stückzahl der Versuchstiere: 240.

Durch diese Behandlung mit dem erfindungsgemäßen Mittel kann im Bereich der Tierernährung die Verwendung von Sonnenblumenextraktionsschrot als Eiweißträger wesentlich erhöht und dadurch können die devisenintensiven Sojaimporte reduziert werden.

**Patentansprüche**

1. Mittel zur Aktivierung fermentativer Prozesse, dadurch gekennzeichnet, daß es neben Konservierungs- bzw. Stabilisierungsmitteln sowie gegebenenfalls Verdünnungsmitteln, Farbstoffen, Geruchskorrigentien und Verdickungsmitteln und dgl. ein oder mehrere Fermente und Saponin enthält.

2. Mittel nach Anspruch 1, dadurch gekennzeichnet, daß es als Saponin ein Triterpen-Saponin enthält.

3. Mittel nach Anspruch 2, dadurch gekennzeichnet, daß es Quillaja-Saponin enthält.

4. Mittel nach einem der Ansprüche 1 bis 3, dadurch gekennzeichnet, daß es als Ferment Glykosidase, Cellulase, Proteinase, Lipase oder Amylase allein oder zusammen mit weiteren Fermenten enthält.

5. Mittel nach einem der Ansprüche 1 bis 4, dadurch gekennzeichnet, daß es 2 g Quillaja-Saponin, 500 g Ferment, 5 g Konservierungsmittel und 300 g Haftmittel pro Liter wässeriger Lösung enthält.

6. Verfahren zum Beschleunigen des fermentativen Abbaues von Naturstoffen, bei welchem den Naturstoffen, insbesondere ligninhaltigen Polysacchariden, Fermente zugesetzt werden, dadurch gekennzeichnet, daß man außerdem Saponin bzw. Sapogenin oder Saponin bzw. Sapogenin enthaltende Extrakte bzw. Präparate in einem Anteil von 0,2 bis 20 g Saponin bzw. Sapogenin pro Tonne Substrat zusetzt.

7. Verfahren nach Anspruch 6, dadurch gekennzeichnet, daß man als Saponin ein Triterpan-Saponin einsetzt.

8. Verfahren nach Anspruch 7, dadurch gekennzeichnet, daß man als Saponin Quillaja-Saponin einsetzt.

9. Verfahren nach einem der Ansprüche 6 bis 8, dadurch gekennzeichnet, daß man als Ferment Cellulase einsetzt.

10. Verfahren nach einem der Ansprüche 6 bis 8, dadurch gekennzeichnet, daß man als Ferment Amylase einsetzt.

11. Verfahren nach einem der Ansprüche 6 bis 8, dadurch gekennzeichnet, daß man als Ferment ein Gemisch aus Amylase, Protease und Lipase einsetzt.

12. Verwendung eines Mittels, welches neben Konservierungs-bzw. Stabilisierungsmitteln sowie gegebenenfalls Verdünnungsmitteln, Farbstoffen, Geruchskorrigentien und Verdickungsmitteln ein oder mehrere Fermente und Saponin enthält, zur Aktivierung fermentativer Prozesse.